# EUROPEAN PATENT APPLICATION

(11) **EP 4 729 502 A1**
(43) Date of publication of application: **22.04.2026**
(21) Application number: 25306721.9
(22) Date of filing: 16.10.2025
(51) Int. Cl.: C07C 7/00, C07C 15/46, C07C 7/04, C08J 11/12, C08J 11/00

(54) **RECOVERY OF STYRENE MONOMER FROM PYROLYSIS OIL**

(30) Priority: 21.10.2024 EP 24306757
(71) Applicant: Technip Energies France, 92741 Nanterre Cedex (FR)
(72) Inventor: VALLIERES, Peter, 92741 Nanterre Cedex (FR); BISHOP, Jonathan, 92741 Nanterre Cedex (FR)
(74) Representative: Edson, Russell Gregory

(57) **Abstract**

A system can be used to reduce the impurities present in a styrene monomer product distilled from a pyrolysis oil. The system can include a fractionation train downstream from a depolymerization unit. The fractionation train may include four fractionation columns in series and produce a styrene monomer product stream comprises at least 99.0 wt% styrene and 300 ppm or less cumene.

## Description

### Technical Field

The present disclosure relates generally to chemical processing and, more particularly (although not necessarily exclusively), to reducing the impurities present in a styrene monomer product distilled from a pyrolysis oil.

### Background

Environmental pollution, resource scarcity, climate change, and the like are contemporary problems that have been driving development of cyclical, or circular, economies to replace various linear economies. For example, processes can be used for recovering raw materials from plastic waste. Plastic waste is increasing and has various negative effects on the environment, on public health, on the economy, and the like. Not all thermoplastic polymers can be recycled at similar rates or efficiencies. Tailoring a chemical recycling process to specific polymers or classes of polymers can be difficult. Additionally, reducing each potential type of impurity with existing systems and techniques can be difficult.

Process for recovering styrene monomer from polystyrene exist. However, it has been found that these processes are typically unable to sufficiently remove cumene to industry standard levels from depolymerization oils that contain higher amounts of the cumene impurity. These processes are therefore unable to produce a styrene monomer product suitable for use in certain downstream applications that require low cumene impurity levels, when treating depolymerization oils containing high amounts of the cumene impurity. The industry standard for many downstream applications of the styrene monomer product is a cumene impurity of 300 ppm (0.03 wt%) or below. The presence of the cumene impurity in higher amounts, such as 700 ppm, in the styrene monomer product renders the product unsuitable for such downstream applications. It has been found that these known processes are typically unable to produce a styrene monomer product having cumene impurity levels at 300 ppm or lower when the depolymerization oil contains higher amounts of the cumene impurity to be removed. The present process has surprisingly been found to produce a styrene monomer product having cumene impurity levels at 300 ppm or lower when the depolymerization oil contains higher amounts of the cumene impurity.

### Summary

In a first aspect there is provided a system comprising: a fractionation train downstream from a depolymerization unit, the fractionation train comprising: a first fractionation column configured to receive a pyrolysis oil from the depolymerization unit and produce a first overhead stream and a first bottoms stream; a second fractionation column configured to receive the first overhead stream and produce a second overhead stream and a second bottoms stream; a third fractionation column configured to receive the second bottoms stream and produce a third overhead stream and a third bottoms stream; and a fourth fractionation column configured to receive the third overhead stream and produce a styrene monomer product stream and a fourth bottoms stream.

One or more embodiments include the system of any previous paragraph, wherein the fourth fractionation column has 2 to 6 beds. Each bed may have 10 to 20 theoretical stages.

One or more embodiments include the system of any previous paragraph, wherein the fourth fractionation column is operated at a bottom temperature from 55°C to 130°C and a top temperature from 35°C to 100°C.

One or more embodiments include the system of any previous paragraph, wherein the fourth fractionation column is operated at a bottom pressure from 15 mmHg to 230 mmHg and a top pressure from 10 mmHg to 150 mmHg.

One or more embodiments include the system of any previous paragraph, wherein the fourth fractionation column contains a condenser for the styrene monomer product stream.

One or more embodiments include the system of any previous paragraph, wherein the system is configured to product a styrene monomer product stream which comprises at least 99.0 wt% styrene and 300 ppm or less cumene, optionally when the pyrolysis oil comprises at least about 0.05 wt% cumene, further optionally when the pyrolysis oil comprises at least about 0.1 wt% cumene, further optionally when the pyrolysis oil comprises at least about 1 wt% cumene and further optionally when the pyrolysis oil comprises no more than about 5 wt% cumene.

One or more embodiments include a process comprising: fractionating a pyrolysis oil comprising styrene and one or more contaminants to produce a first overhead stream and a first bottoms stream, wherein the one or more contaminants comprise C9+ aromatic compounds, wherein the first bottoms stream is enriched in C9+ aromatic compounds compared to the pyrolysis oil; fractionating the first overheads stream to produce a second overhead stream and a second bottoms stream, wherein the second bottoms stream is enriched in styrene and C9+ aromatic compounds compared to the first overheads stream; fractionating the second bottoms stream to produce a third overhead stream and a third bottoms stream, wherein the third overhead stream is enriched in styrene compared to the second bottoms stream; and fractionating the third overhead stream to produce a styrene monomer product stream and a fourth bottoms stream.

One or more embodiments include the process of any previous paragraph, further comprising: condensing the styrene monomer product stream; and combining a portion of the condensed styrene monomer product stream with the third overhead stream with a ratio of the condensed styrene monomer product stream to the third overhead stream being 5 to 10.

One or more embodiments include the process of any previous paragraph, wherein the fractionating of the third overhead stream is performed in a fourth fractionation column operated at a bottom temperature from 55°C to 130°C and a top temperature from 35°C to 100°C.

One or more embodiments include the process of any previous paragraph, wherein the fractionating of the third overhead stream is performed in a fourth fractionation column operated at a bottom pressure from 15 mmHg to 230 mmHg and a top pressure from 10 mmHg to 150 mmHg.

One or more embodiments include the process of any previous paragraph, wherein the fractionating of the third overhead stream is performed in a fourth fractionation column having 2 to 6 beds, and wherein each bed has 10 to 20 theoretical stages.

One or more embodiments include the process of any previous paragraph, wherein the fractionating of the third overhead stream is performed in a fourth fractionation column configured to feed the third overhead stream into the fourth fractionation column at a location having at least two beds above and at least two beds below the location.

One or more embodiments include the process of any previous paragraph, wherein the styrene monomer product stream comprises at least 99.0 wt% styrene and 300 ppm or less cumene, optionally wherein the styrene monomer product stream comprises at least 99.8 wt% styrene and 300 ppm or less cumene.

One or more embodiments include the process of any previous paragraph, further comprising:

combining two or more of the first bottoms stream, the third bottoms stream, and the fourth bottoms stream.

One or more embodiments include the process of any previous paragraph, wherein the pyrolysis oil is produced by depolymerization of a waste plastics stream comprising at least 75 wt% styrenic polymer, based on a total weight of the waste plastics stream, and/or wherein the pyrolysis oil comprises at least about 0.05 wt% cumene, optionally at least about 0.1 wt% cumene, further optionally at least about 1 wt% cumene, and further optionally wherein the pyrolysis oil comprises no more than about 5 wt% cumene.

In another aspect, there is provided a process for producing a styrene monomer product, wherein the process comprises performing the process according to any previous paragraph.

Surprisingly, the use of a fourth fractionation column in the system of the present disclosure or the fourth fractionating step in the process of the present disclosure has been found to reduce the amount of cumene present in the styrene monomer product stream, even when the treated pyrolysis oil comprises higher amounts of the cumene impurity.

### Brief Description of the Drawings

FIG. 1 is a diagram of a styrene processing system for generating a styrene monomer product stream according to some examples of the present disclosure.
FIG. 2 is a diagram of a purification process within a styrene processing system according to some examples of the present disclosure.
FIG. 3 is a flowchart of a process for reducing impurities in a styrene monomer product using fractionation according to some examples of the present disclosure.

### Detailed Description

Certain aspects and features of the present disclosure relate to recovering high-purity styrene (also referred to as "styrene monomer") from the pyrolysis oil produced by depolymerizing styrene. The depolymerization process may include a pyrolysis process or other suitable depolymerization process that produces pyrolysis oil from waste plastics, such as styrenic waste. The pyrolysis oil may include the majority of the styrene produced in the depolymerization process and contaminants, including contaminants with a similar boiling point to styrene like cumene and o-xylene. The processes and systems of the present disclosure may include a fractionation train to separate the styrene monomer from various impurities included in the pyrolysis oil including similar boiling point contaminants. Advantageously, the produced styrene monomer product may have 300 ppm or less of cumene.

The illustrative examples herein are given to introduce the reader to the general subject matter discussed here and are not intended to limit the scope of the disclosed concepts. The following sections describe various additional features and examples with reference to the drawings in which like numerals indicate like elements but, like the illustrative examples, should not be used to limit the present disclosure.

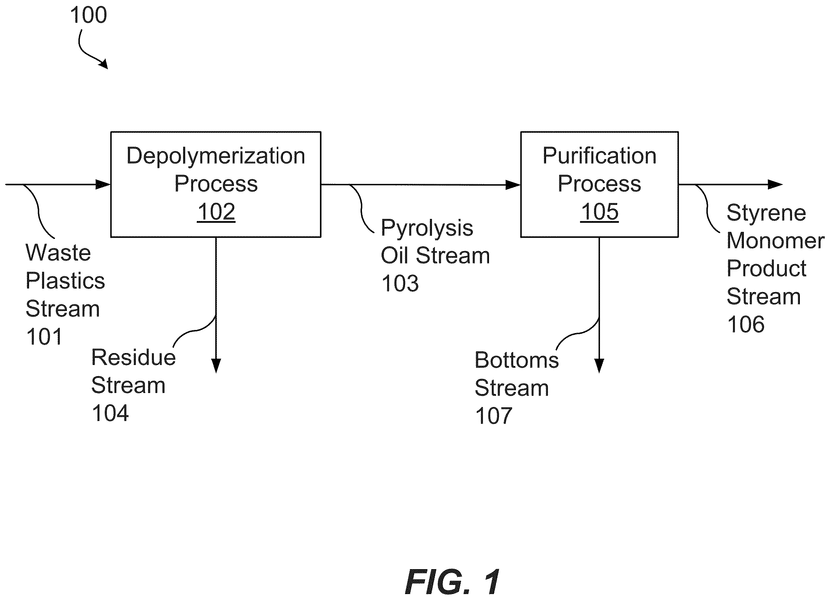

FIG. 1 illustrates a process flow diagram of a styrene processing system 100 for generating a styrene monomer product stream 106 according to at least some examples of the present disclosure. The styrene processing system 100 may include a depolymerization process 102 that depolymerizes a waste plastics stream 101 and separates the product into a pyrolysis oil stream 103 and a residue stream 104.

The waste plastics stream 101 may have a styrenic polymer content of at least 75 wt% (e.g., 75 wt% to 100 wt%, 80 wt% to 100 wt%, or 90 wt% to 100 wt%), based on a total weight of the waste plastics stream 101. The waste plastics stream 101 may have a polystyrene content of at least 75 wt% (e.g., 75 wt% to 100 wt%, 80 wt% to 100 wt%, or 90 wt% to 100 wt%), based on a total weight of the waste plastics stream 101.

The depolymerization process 102 may include depolymerizing waste plastics containing styrenic polymers in a pyrolysis reactor.

The pyrolysis oil stream 103 may be the main product of the depolymerization process. The pyrolysis oil stream 103 may contain most of the styrene produced in the depolymerization process. The pyrolysis oil stream 103 may include styrene at a concentration ranging from 50 wt% to 80 wt% (e.g., 50 wt% to 65 wt%, 55 wt% to 75 wt%, or 60 wt% to 80 wt%). The pyrolysis oil may further contain compounds that may include, but are not limited to, benzene, toluene, ethylbenzene, alpha-methylstyrene, cumene, xylenes, compounds lighter than benzene, compounds heavier than alpha-methylstyrene, and any combination thereof. Optionally, a polymerization inhibitor, such as a mono- or dinitro-aromatic compound, may be added to the pyrolysis oil stream 103 before purification and/or a stream within the purification process 105.

In certain embodiments of the process and system, the pyrolysis oil comprises cumene in an amount of at least about 0.05 wt%, or at least about 0.1 wt%, or at least about 0.5 wt%, or at least about 1 wt%, or at least about 1.5 wt%. In certain embodiments of the process and system, the pyrolysis oil comprises cumene in an amount of less than about 10 wt%, or less than about 5 wt%, or less than about 3 wt%, or less than about 2 wt%. In certain embodiments of the process and system, the pyrolysis oil comprises cumene in an amount of between about 0.05 wt% to about 2, 3, 4, 5 or 10 wt%, or between about 0.1 wt% to about 2, 3, 4, 5 or 10 wt%, or between about 0.5 wt% to about 2, 3, 4, 5 or 10 wt%, or between about 1 wt% to about 2, 3, 4, 5 or 10 wt%, or between about 1.5 wt% to about 2, 3, 4, 5 or 10 wt% The phrase "higher amounts of the cumene impurity", and similar phrases, used herein refers to a pyrolysis oil comprising cumene in the amounts specified in this paragraph.

The residue stream 104 may include heavy, non-volatile material.

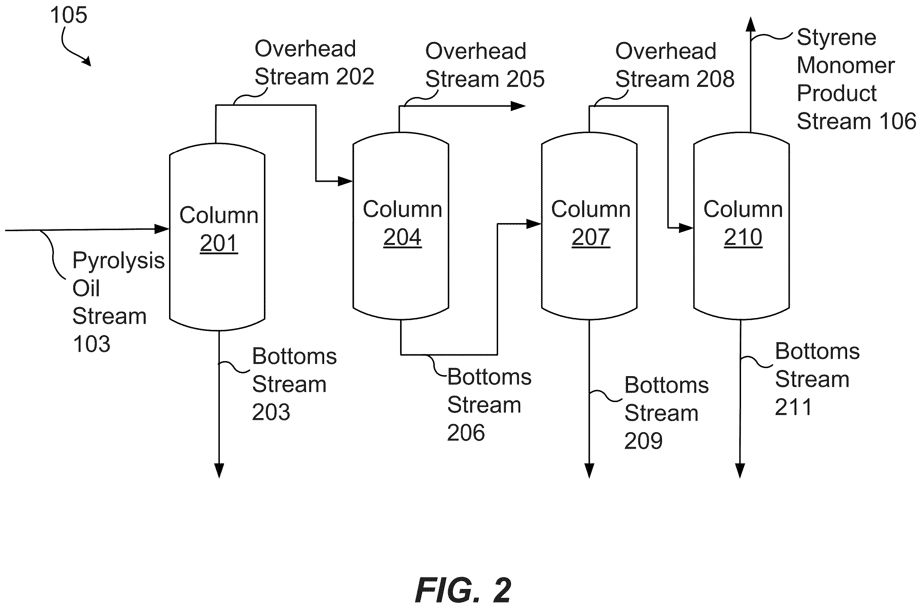

The pyrolysis oil stream 103 may then be purified by purification process 105 to produce a styrene monomer product stream 106 and a bottoms stream 107. FIG. 2, with continued reference to FIG. 1, illustrates a process flow diagram of the purification process 105 according to at least some examples of the present disclosure. In the purification process 105, the pyrolysis oil stream 103 is processed through a fractionation train that includes four fractionation columns 201, 204, 207, and 210 to produce the styrene monomer product stream 106.

The pyrolysis oil stream 103 may be separated in a first fractionation column 201 and separated into a first overhead stream 202 and a first bottoms stream 203. The first bottoms stream 203 may be enriched in C₉₊ aromatic compounds as compared to the pyrolysis oil stream 103.

The first fractionation column 201 may be a reboiled stripper, which may be a column that includes a reboiler for the liquid column bottoms but not a condenser for the vapor overhead. The first bottoms stream 203 may contain part of the C₉₊ aromatic compounds, and preferably virtually all of the C₁₀₊ aromatic compounds, in the feed. The first overhead stream 202 may consist of the remainder of the feed. In embodiments, the first fractionation column 201 separates a sufficient portion of the divinylbenzene in the depolymerization oil feed from most of the styrene so that crosslinking of polymer is not a significant problem in the downstream columns. A majority of the divinylbenzene may leave first fractionation column 201 as part of the first bottoms fraction, and most of the styrene may leave the first fractionation column 201 in the overhead vapor.

The first fractionation column 201 typically has between 1 and 15 theoretical stages, such as from 5 to 10 theoretical stages, with the pyrolysis oil stream 103 feed being introduced into the first fractionation column 201 at or close to the uppermost stage. Although the internals of the first fractionation column 201 can include fractionation packing, it is preferable to employ distillation trays since these are less expensive and easier to clean of insoluble polymer build-up than packing.

The first fractionation column 201 may be operated at (i) a bottom temperature from 90°C to 155°C (e.g., 100°C to 150°C), and a top or head temperature from 55°C to 100°C (e.g., 65°C to 90°C), (ii) a bottom pressure from 40 mmHg to 300 mmHg (e.g., 70 mmHg to 200 mmHg), and (iii) a top or head pressure from 40 mmHg to 180 mmHg (e.g., 50 mmHg to 120 mmHg). The first fractionation column 201 may include a reboiler to redistill part of the first bottoms fraction. However, the first fractionation column 201 may not include a side cut for separating the ethylbenzene and styrene monomer from the lighter components and preferably does not have a condenser and associated pump for refluxing part of the overhead fraction.

The first overhead stream 202 may be further separated in a second fractionation column 204 into a second overhead stream 205 and a second bottoms stream 206. The second overhead stream 205 may be enriched in benzene, toluene, and ethylbenzene as compared to the first overhead stream 202. The second bottoms stream 206 may be enriched in styrene and C₉₊ aromatic compounds (e.g., cumene) as compared to the first overhead stream 202.

The second fractionation column 204 may include distillation trays but typically is a packed column. The second fractionation column 204 may have at least 20 theoretical stages, such as from 20 to 120 theoretical stages (e.g., 40 to 100 theoretical stages). When the first fractionation column 201 does not include reflux from the condensed first column overhead, essentially all the heat put into the first fractionation column 201 is in the vapor sent to the second fractionation column 204 leading to an overall improvement in energy efficiency relative to a column system with a condenser and reflux.

The second fractionation column 204 may be a complete distillation system with a reboiler for the liquid column bottoms and a condenser for the vapor column overhead. Typically, the second fractionation column 204 may be operated at (i) a bottom temperature from 60 to 120°C (e.g., 70°C to 105°C), (ii) a top or head temperature from 35°C to 100°C (e.g., 40°C to 70°C), (iii) a bottom pressure from 45 mmHg to 370 mmHg (e.g., from 65 mmHg to 210 mmHg), and (iv) a top or head pressure from 30 mmHg to 290 mmHg (e.g., from 40 mmHg to 145 mmHg). Under these conditions, the second fractionation column 204 is effective to divide the first overhead stream 202 into a second overhead stream 205 rich in benzene, toluene, and ethylbenzene compared to the first overhead stream 202, and a second bottoms stream 206 rich in styrene monomer and C₉₊ aromatic compounds compared to the first overhead stream 202.

The second overhead stream 205 exiting the second fractionation column 204 may be passed through a condenser where the majority of the second overhead stream 205 is condensed. Part of the resultant condensate may then be returned to the second fractionation column 204 as reflux, while some or all of the remainder is recovered as a byproduct (distillate) stream, which may be a feed to an aromatics plant, with the ratio of reflux to distillate typically lying between 15 to 30. Part of the condensate may be fed back to the top of the first fractionation column 201 as a reflux stream, with the ratio of condensate fed back to the first fractionation column 201 to the distillate stream is typically from 0.2 and 0.4. Any water in the pyrolysis oil stream 103 will mostly condense in the condenser and form a separate liquid phase. Optionally, this liquid water can be separated off from the liquid hydrocarbon.

The second bottoms stream 206 from the second fractionation column 204 may be composed mainly of styrene monomer and C₉₊ aromatic compounds. To maximize product separation in the second fractionation column 204, a portion of the second bottoms stream 206 may be fed to a reboiler, heated and then returned to the bottom of the second fractionation column 204.

The second bottoms stream 206 may be further separated in a third fractionation column 207 into a third overhead stream 208 and a third bottoms stream 209. The second bottoms stream 206 may be supplied to the third fractionation column 207 optionally together with additional polymerization inhibitor. The third overhead stream 208 may be enriched in styrene and similar boiling point compounds like cumene as compared to the second bottoms stream 206. The third bottoms stream 209 may be enriched in C₉₊ aromatic compounds as compared to the second bottoms stream 206.

The third fractionation column 207 may include distillation trays but typically is a packed column. Generally, the third fractionation column has from 10 to 120 theoretical stages (e.g., 30 to 100 theoretical stages). The third fractionation column 207 may be a complete distillation system with a reboiler for the third bottoms stream 209 and a condenser for the third overhead stream 208. Typically, the third fractionation column 207 is operated at (i) a bottom temperature from 55°C to 130°C (e.g., 75°C to 115°C), (ii) a top or head temperature from 35°C to 100°C (e.g., 40°C to 80°C), (iii) a bottom pressure from 15 mmHg to 230 mmHg (e.g., 35 mmHg to 160 mmHg), and (iv) a top or head pressure from 10 mmHg to 150 mmHg (e.g., from 15 mmHg to 90 mmHg).

The third overhead stream 208 exiting the third fractionation column 207 may be passed through a condenser where the majority of the third overhead fraction is typically condensed. Part of the resultant condensate may then be returned to the third fractionation column 207 as reflux, with the ratio of reflux to condensate typically lying between 2.5 to 6.

The third overhead stream 208 may be further separated in a fourth fractionation column 210 into the styrene monomer product stream 106 and a fourth bottoms stream 211. The styrene monomer product stream 106 may be enriched in styrene as compared to the third overhead stream 208. The fourth bottoms stream 211 may be enriched in cumene as compared to the third overhead stream 208.

The fourth fractionation column 210 may include distillation trays but typically is a packed column. Generally, the third fractionation column has from 10 to 120 theoretical stages (e.g., 30 to 100 theoretical stages) (e.g., 2 to 6 beds each with 10 to 20 theoretical stages, or 3 to 5 beds each with 10 to 15 theoretical stages). The third overhead stream 208 may be fed into the fourth fractionation column 210 at around a midpoint of the fourth fractionation column 210 (e.g., with an equal number of beds above and below the introduction point).

The fourth fractionation column 210 may be a complete distillation system with a reboiler for the fourth fractionation column 210 and a condenser for the styrene monomer product stream 106. Typically, the fourth fractionation column 210 is operated at (i) a bottom temperature from 55°C to 130°C (e.g., 75°C to 115°C), (ii) a top or head temperature from 35°C to 100°C (e.g., 40°C to 80°C), (iii) a bottom pressure from 15 mmHg to 230 mmHg (e.g., 35 mmHg to 160 mmHg), and (iv) a top or head pressure from 10 mmHg to 150 mmHg (e.g., from 15 mmHg to 90 mmHg).

The styrene monomer product stream 106 exiting the fourth fractionation column 210 may be passed through a condenser where the majority of the styrene monomer product stream 106 is typically condensed. Part of the resultant condensate may then be returned to the fourth fractionation column 210 as reflux, with the ratio of reflux to condensate (i.e., condensed fourth overhead stream) typically lying between 5 to 10 (e.g., 6 to 8). The remainder is recovered as the desired styrene monomer product stream 106, which may have a purity of at least 99.0 wt% (e.g., 99.5 wt% or greater, 99.8 wt% or greater, 99.9 wt% or greater), based on a total weight of the styrene monomer product stream 106.

The styrene monomer product stream 106 may have a cumene concentration of 300 ppm or less (e.g., 250 ppm or less, 200 ppm or less, 0 ppm to 300 ppm, 10 ppm to 300 ppm, 0 ppm to 250 ppm, 10 ppm to 250 ppm, or 0 ppm to 200 ppm, 10 ppm to 200 ppm). The styrene monomer product stream 106 may have a total concentration of contaminants (e.g., cumene, o-xylene, and others) of 1.0% or less (e.g., 0.5 wt% or greater, 0.2 wt% or greater, 0.1 wt% or greater), based on a total weight of the styrene monomer product stream 106.

Any combination of the first bottoms stream 203, the third bottoms stream 209, and the fourth bottoms stream 211 may be combined and purged from the system as the bottom stream 107 of FIG. 1.

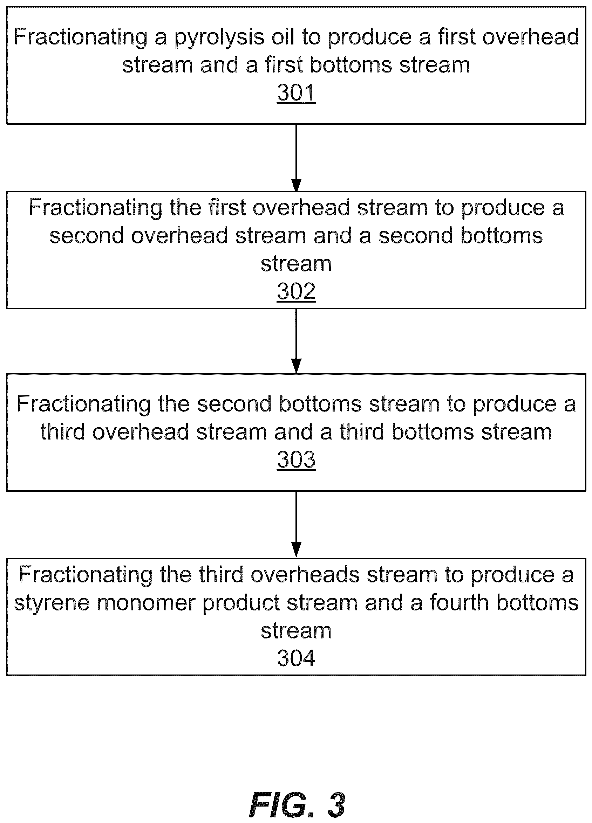

FIG. 3 is a flowchart of a process for reducing impurities in a styrene monomer product using fractionation according to some examples of the present disclosure. In step 301, the process includes fractionating a pyrolysis oil to produce a first overhead stream and a first bottoms stream. Then, in step 302, the process includes fractionating the first overhead stream to produce a second overhead stream and a second bottoms stream. In step 303, the process includes fractionating the second bottoms stream to produce a third overhead stream and a third bottoms stream. Then, in step 304, the process includes fractionating the third overheads stream to produce a styrene monomer product stream and a fourth bottoms stream. The composition of each of the streams are consistent with the disclosure relative to FIGS. 1-2. Further, steps like condensing any of the overhead streams may optionally be included in the process as described in the disclosure relative to FIGS. 1-2.

### Example

An embodiment according to the four-column distillation process described herein (and as shown in Figure 2) is compared to a process using a three-column distillation as described in WO 2024/015227 A1 (WO '227) . The depolymerization oil is a depolymerization oil containing higher amounts of the cumene impurity than described in WO '227. The composition of the depolymerization oil is shown in Table 1 below. As can be seen from Table 1, the depolymerization oil contains 1.8 wt% of cumene. Table 1 also shows the composition of the styrene product that will be obtained from the three-column distillation according to WO '227 and the four-column distillation according to an embodiment of the present process.

**Table 1**

| | High-Cumene Depolymerization oil (wt. %) | Styrene Product from Three-Column distillation (wt. %) | Styrene Product from Four-Column distillation (wt. %) |
|---|---|---|---|
| Toluene | 14.2 | nil | nil |
| Ethylbenzene (EB) | 10.0 | 0.02 | 0.02 |
| Xylenes | 0.09 | 0.04 | 0.04 |
| Styrene | 64.0 | 99.8 | 99.8 |
| Phenylacetylene | 0.09 | 0.07 | 0.07 |
| Cumene | 1.8 | 0.07 | 0.03 |
| α-Methylstyrene | 9.1 | nil | nil |
| Other C₉-C₁₀ | 0.18 | nil | nil |
| C₁₁₊ components | 0.54 | nil | nil |

As can be seen from Table 1, the three-column distillation fails to reduce the amount of cumene present in the styrene product to industry standard levels for many downstream applications (i.e., 300 ppm or below). However, the four-column distillation can reduce the amount of cumene present in the styrene product to industry standard levels for many downstream applications (i.e., 300 ppm or below). This shows that the four-column system and process of the present disclosure can reduce cumene impurity levels to sufficient levels in the styrene product when the depolymerization oil contains higher amounts of the cumene impurity. This was a surprising finding since, as can be seen from Table 1, the addition of the fourth distillation step will not have any real impact on the amount of the other components of the styrene production composition.

The foregoing description of certain examples, including illustrated examples, has been presented only for the purpose of illustration and description and is not intended to be exhaustive or to limit the disclosure to the precise forms disclosed. Numerous modifications, adaptations, and uses thereof will be apparent to those skilled in the art without departing from the scope of the disclosure. For instance, any examples described herein can be combined with any other examples to yield further examples.

The disclosure is also defined according to the following numbered embodiments.
1. A system comprising:
   a fractionation train downstream from a depolymerization unit, the fractionation train comprising:
   a first fractionation column configured to receive a pyrolysis oil from the depolymerization unit and produce a first overhead stream and a first bottoms stream;
   a second fractionation column configured to receive the first overhead stream and produce a second overhead stream and a second bottoms stream;
   a third fractionation column configured to receive the second bottoms stream and produce a third overhead stream and a third bottoms stream; and
   a fourth fractionation column configured to receive the third overhead stream and produce a styrene monomer product stream and a fourth bottoms stream, wherein the styrene monomer product stream comprises at least 99.0 wt% styrene and 300 ppm or less cumene.
2. The system of embodiment 1, wherein the fourth fractionation column has 2 to 6 beds.
3. The system of embodiment 2, wherein each bed has 10 to 20 theoretical stages.
4. The system of embodiment 1, wherein the fourth fractionation column is operated at a bottom temperature from 55°C to 130°C and a top temperature from 35°C to 100°C.
5. The system of embodiment 1, wherein the fourth fractionation column is operated at a bottom pressure from 15 mmHg to 230 mmHg and a top pressure from 10 mmHg to 150 mmHg.
6. The system of embodiment 1, wherein the fourth fractionation column contains a condenser for the styrene monomer product stream.
7. A process comprising:
   fractionating a pyrolysis oil comprising styrene and one or more contaminants to produce a first overhead stream and a first bottoms stream, wherein the one or more contaminants comprise C9+ aromatic compounds, wherein the first bottoms stream is enriched in C9+ aromatic compounds compared to the pyrolysis oil;
   fractionating the first overheads stream to produce a second overhead stream and a second bottoms stream, wherein the second bottoms stream is enriched in styrene and C9+ aromatic compounds compared to the first overheads stream;
   fractionating the second bottoms stream to produce a third overhead stream and a third bottoms stream, wherein the third overhead stream is enriched in styrene compared to the second bottoms stream; and
   fractionating the third overhead stream to produce a styrene monomer product stream and a fourth bottoms stream, wherein the styrene monomer product stream comprises at least 99.0 wt% styrene and 300 ppm or less cumene.
8. The process of embodiment 7 further comprising:
   condensing the styrene monomer product stream; and
   combining a portion of the condensed styrene monomer product stream with the third overhead stream with a ratio of the condensed styrene monomer product stream to the third overhead stream being 5 to 10.
9. The process of embodiment 7, wherein the fractionating of the third overhead stream is performed in a fourth fractionation column operated at a bottom temperature from 55°C to 130°C and a top temperature from 35°C to 100°C.
10. The process of embodiment 7, wherein the fractionating of the third overhead stream is performed in a fourth fractionation column operated at a bottom pressure from 15 mmHg to 230 mmHg and a top pressure from 10 mmHg to 150 mmHg.
11. The process of embodiment 7, wherein the fractionating of the third overhead stream is performed in a fourth fractionation column having 2 to 6 beds, and wherein each bed has 10 to 20 theoretical stages.
12. The process of embodiment 7, wherein the fractionating of the third overhead stream is performed in a fourth fractionation column configured to feed the third overhead stream into the fourth fractionation column at a location having at least two beds above and at least two beds below the location.
13. The process of embodiment 7, wherein the styrene monomer product stream comprises at least 99.8 wt% styrene and 300 ppm or less cumene.
14. The process of embodiment 7 further comprising:
   combining two or more of the first bottoms stream, the third bottoms stream, and the fourth bottoms stream.
15. The process of embodiment 7, wherein the pyrolysis oil is produced by depolymerization of a waste plastics stream comprising at least 75 wt% styrenic polymer, based on a total weight of the waste plastics stream.

## Claims

1. A system comprising:
a fractionation train downstream from a depolymerization unit, the fractionation train comprising:
a first fractionation column configured to receive a pyrolysis oil from the depolymerization unit and produce a first overhead stream and a first bottoms stream;
a second fractionation column configured to receive the first overhead stream and produce a second overhead stream and a second bottoms stream;
a third fractionation column configured to receive the second bottoms stream and produce a third overhead stream and a third bottoms stream; and
a fourth fractionation column configured to receive the third overhead stream and produce a styrene monomer product stream and a fourth bottoms stream.

2. The system of claim 1, wherein the fourth fractionation column has 2 to 6 beds, optionally wherein each bed has 10 to 20 theoretical stages.

3. The system of any preceding claim, wherein the fourth fractionation column is operated at a bottom temperature from 55°C to 130°C and a top temperature from 35°C to 100°C.

4. The system of any preceding claim, wherein the fourth fractionation column is operated at a bottom pressure from 15 mmHg to 230 mmHg and a top pressure from 10 mmHg to 150 mmHg.

5. The system of any preceding claim, wherein the fourth fractionation column contains a condenser for the styrene monomer product stream.

6. The system of any preceding claim, wherein the system is configured to produce a styrene monomer product stream which comprises at least 99.0 wt% styrene and 300 ppm or less cumene, optionally when the pyrolysis oil comprises at least about 0.05 wt% cumene, further optionally when the pyrolysis oil comprises at least about 0.1 wt% cumene, further optionally when the pyrolysis oil comprises at least about 1 wt% cumene and further optionally when the pyrolysis oil comprises no more than about 5 wt% cumene.

7. A process comprising:
fractionating a pyrolysis oil comprising styrene and one or more contaminants to produce a first overhead stream and a first bottoms stream, wherein the one or more contaminants comprise C₉₊ aromatic compounds, wherein the first bottoms stream is enriched in C₉₊ aromatic compounds compared to the pyrolysis oil;
fractionating the first overheads stream to produce a second overhead stream and a second bottoms stream, wherein the second bottoms stream is enriched in styrene and C₉₊ aromatic compounds compared to the first overheads stream;
fractionating the second bottoms stream to produce a third overhead stream and a third bottoms stream, wherein the third overhead stream is enriched in styrene compared to the second bottoms stream; and
fractionating the third overhead stream to produce a styrene monomer product stream and a fourth bottoms stream.

8. The process of claim 7 further comprising:
condensing the styrene monomer product stream; and
combining a portion of the condensed styrene monomer product stream with the third overhead stream with a ratio of the condensed styrene monomer product stream to the third overhead stream being 5 to 10.

9. The process of claim 7 or claim 8, wherein the fractionating of the third overhead stream is performed in a fourth fractionation column operated at a bottom temperature from 55°C to 130°C and a top temperature from 35°C to 100°C.

10. The process of any of claims 7 to 9, wherein the fractionating of the third overhead stream is performed in a fourth fractionation column operated at a bottom pressure from 15 mmHg to 230 mmHg and a top pressure from 10 mmHg to 150 mmHg.

11. The process of any of claims 7 to 10, wherein the fractionating of the third overhead stream is performed in a fourth fractionation column having 2 to 6 beds, and wherein each bed has 10 to 20 theoretical stages.

12. The process of any of claims 7 to 11, wherein the fractionating of the third overhead stream is performed in a fourth fractionation column configured to feed the third overhead stream into the fourth fractionation column at a location having at least two beds above and at least two beds below the location.

13. The process of any of claims 7 to 12, wherein the styrene monomer product stream comprises at least 99.0 wt% styrene and 300 ppm or less cumene, optionally wherein the styrene monomer product stream comprises at least 99.8 wt% styrene and 300 ppm or less cumene.

14. The process of any of claims 7 to 13 further comprising:
combining two or more of the first bottoms stream, the third bottoms stream, and the fourth bottoms stream.

15. The process of any of claims 7 to 14, wherein the pyrolysis oil is produced by depolymerization of a waste plastics stream comprising at least 75 wt% styrenic polymer, based on a total weight of the waste plastics stream; and/or
wherein the pyrolysis oil comprises at least about 0.05 wt% cumene, optionally at least about 0.1 wt% cumene, further optionally at least about 1 wt% cumene, and further optionally wherein the pyrolysis oil comprises no more than about 5 wt% cumene.
